# EUROPEAN PATENT APPLICATION

(11) **EP 0 615 728 A2**
(43) Date of publication of application: **21.09.1994**
(21) Application number: 94200380.7
(22) Date of filing: 14.02.1994
(51) Int. Cl.: A61B 17/58, A61F 2/30

(54) **Orthopaedic reconstruction plate**

(30) Priority: 16.02.1993 US 16840; 21.04.1993 US 50857; 18.08.1993 US 108997
(71) Applicant: MIKHAIL, Michael W.E., Toledo, OH 43623 (US)
(72) Inventor: MIKHAIL, Michael W.E., Toledo, OH 43623 (US)
(74) Representative: Mays, Julie

(57) **Abstract**

An orthopaedic reconstruction plate (10) designed to be secured to the femur to contain bone graft material placed in the area of the lesser trochanter includes a series of pins extending from the inner surface facing the bone to prevent excessive compressive forces from being imparted to the bone. The plate may have a series of perforations to enhance the flow of blood to the bone. The plate may also include a plurality of sections and an area of reduced thickness between said sections in order to be bendable.

## Description

### Cross Reference To Related Application

This application is a continuation-in-part of my co-pending U.S. application, Serial No. 08/050,857, filed April 21, 1993 which is a continuation-in-part of Serial No. 08/016,840, filed February 16, 1993.

### Background Art

The present invention relates to orthopaedic hip surgery and is specifically directed to a method and device for use in building up the proximal femur in the trochanteric region. It is particularly useful in hip replacement surgery in which a femoral stem prosthesis is implanted in the intramedullary canal of the femur. Many patients requiring hip revision surgery have significant bone deficiency particularly in the supratrochanteric area adjacent the lesser trochanter. In those patients, the orthopaedic surgeon will utilize bone graft material in an attempt to reconstitute the bone in that area sufficiently to provide a sound support for the prosthesis. Additionally, the reconstituted bone assists in maintaining proper length of the femur following implantation of the prosthesis. However, it is frequently difficult to retain the bone graft material in the specific locations desired.

### Disclosure of Invention

The present invention is directed to a hip revision plate specifically designed to support bone graft material in the area of the proximal femur and more particularly that portion of the proximal femur in the area of the lesser trochanter. The plate is designed of a flexible surgical grade material such as malleable metal which may be wrapped around and fastened to the femur in the area of the lesser trochanter and the greater trochanter. The plate is provided with a plurality of apertures for receiving screws for affixing the revision plate to the femur. A plurality of pins or posts extend from the side of the plate intended to face the bone for the purpose of preventing excessive compressive forces being imparted to the bone upon fastening with screws. Excessive compressive forces imparted to the bone can interfere with the blood supply to the bone. Under one embodiment the plate is provided with a tab or a tail which may be folded around a hinge line parallel to the longitudinal axis to engage and be screwed to the lesser trochanter. Under another embodiment, the plate is provided with wires and/or integral strips which may be wrapped around the femur to provide additional support.

The plate may be used in combination with biocompatible mesh which is placed directly over the defect and the surrounding bone, between the bone and the plate. The biocompatible mesh may be a surgical grade of Dacron® or a malleable, surgical grade metal.

The plate and the mesh may also be used in repairing measured longitudinal or vertical femoral defects to contain the allograft or bone material before its compaction.

A method of applying and supporting bone graft material utilizing the orthopaedic reconstruction plate of the present invention is also disclosed.

Accordingly, it is an object of the present invention to provide an orthopaedic reconstruction plate specifically tailored to support bone graft material in the proximal femur or in a long bone.

It is a further object of the present invention to provide a method of building up the proximal femur in the trochanteric region with bone graft material utilizing the orthopaedic reconstruction plate of the present invention, building the femur from the inside with crushed cancellous allograft.

### Brief Description of the Drawings

Fig. 1 is an elevational view of a femur showing a femoral hip joint prosthesis implanted in the intramedullary canal and showing one embodiment of the orthopaedic reconstruction plate of the present invention fixed in place around the greater and lesser trochanter.

Figs 2 and 3 are views similar to Fig. 1 showing other embodiments of the orthopaedic reconstruction plate of the present invention fixed in place.

Fig. 4 is a plan view of the inner side of the orthopaedic reconstruction plate of the present invention intended to face toward and engage the femur.

Fig. 5 is a perspective view showing a fragmentary length of the orthopaedic reconstruction plate.

Fig. 6 is a sectional view taken through line 6-6 of Fig. 4.

Fig. 7 is a sectional view taken through line 7-7 of Fig. 4.

Fig. 8 is a view similar to Fig. 7 showing a different type of means for attaching the wires to the tab or other portions of the orthopaedic reconstruction plate.

Fig. 9 is view similar to Fig. 5 showing a different type of pin extending from the inner surface of the orthopaedic reconstruction plate.

Figs. 10-12 are plan views of other embodiments of the orthopaedic reconstruction plate of the present invention.

Fig. 13 is a sectional view taken through line 13-13 of Fig. 12.

Fig. 14 is a sectional view showing a hip joint prosthesis following implantation with one orthopaedic reconstruction plate utilized for retaining bone graft material in the proximal femur in the trochanteric region and a second orthopaedic reconstruction plate utilized for retaining bone graft material in a defect in the femoral cortices in another area of the femur.

Fig. 15 is a fragmentary view of a section of a femur having a larger defect and a different embodiment of orthopaedic reconstruction plate for retaining bone graft material.

Fig. 16 is a sectional view taken through line 16-16 of Fig. 14.

Fig. 17 is a fragmentary elevational view of a long bone such as a femur showing a modified embodiment of the present invention.

Fig. 18 is a sectional view taken through line 18-18 of Fig. 17.

Fig. 19 is a view similar to Fig. 17 showing yet another embodiment.

Fig. 20 is a sectional view taken through line 20-20 of Fig. 19.

Fig. 21 is an elevational view of a femur showing a femoral hip joint prosthesis implanted in the intramedullary canal, with such femur having a longitudinal or vertical defect.

Fig. 22 is a view similar to Fig. 21 showing another embodiment of the present invention in position for repairing such defect.

Fig. 23 is an elevational view of a femur showing a femoral hip joint prosthesis implanted in the intramedullary canal and showing yet another embodiment of orthopaedic reconstruction plate fixed in place around the greater and less trochanter.

Fig. 24 is a plan view of the outer side of the reconstruction plate of the embodiment of Fig. 23.

Fig. 25 is an enlarged fragmentary view of one section and a portion of adjacent sections of the embodiment of Fig. 23.

Fig. 26 is a sectional view taken through line 26-26 of Fig. 25.

### Best Mode of Carrying Out Invention

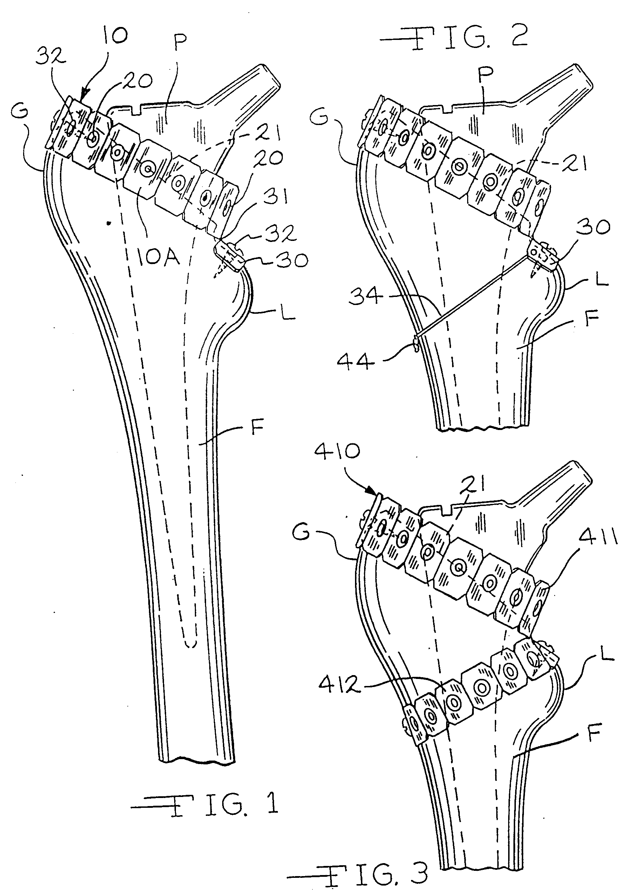

Referring now to Figs. 1 and 4-7, there is provided a orthopaedic reconstruction plate generally designated by the numeral 10. The plate 10 is preferably formed of malleable metal suitable for implantation in the human body such as stainless steel or a cobalt-chrome-molybdenum alloy such as that manufactured and sold by Zimmer Company, Warsaw, Indiana, under its trademark ZIMALOY®. However, it is within the contemplation of the present invention that the hip revision plate be manufactured from a suitable biocompatible plastic. The plate has a thickness in the range of 1 to 2 mm and can readily be wrapped around the bone to provide a contour approximately matching the contour of the bone. The plate 10 has an inner surface 12 intended to face the bone when engaged to the femur F, an outer surface 14, an upper edge 16 and a lower edge 18. The plate 10 is provided with a series of holes 20 extending generally along the Iongitudinal axis A of the plate. The holes are countersunk as shown at 22 in Fig. 6.

The plate 10 should be sufficiently long to be wrapped substantially around the proximal end of the femur F of a patient in the area of the greater trochanter G and lesser trochanter L in order to permit the surgeon to build up the level of deficient bone which is shown as line 21 in Figs. 1-3. A length on the order of 140 mm is preferred. However, since the required length will vary depending upon the size of the patient, it may be desirable to have the plate 10 formed in a length longer than required and to have the capability of trimming it to the desired length during the surgical procedure. If desired, the plate could be wound in a coil having an overall length sufficient for multiple surgeries. The breadth of the plate should be in the range of 9 to 15 mm.

A plurality of pins 24 extend from the inner surface 12 of the plate 10 intended to face the bone. The pins are approximately 1-3 mm in length and have a diameter of 1-2 mm at the base portion adjacent the inner surface 12. Preferably each of the pins 24 tapers to a free end 26. The free end 26 may be rounded but is preferably blunt so that it does not readily penetrate the adjacent bone material. The purpose of the pins 24 is to prevent excessive and damaging compressive forces being imparted to the bone which may interfere with blood supply to the femur F, particularly if the plate 10 completely encircles the bone.

The plate 10 has a series of laterally extending lines 28 of reduced thickness which divide the plate into a series of sections 10A. As will be appreciated, the length of the plate 10 can be readily shortened during surgery by simply removing one or more of the sections 10A by cutting along one of the lines 28 of reduced thickness or by repeatedly folding it along one of such lines until it is broken.

Integrally formed with one of the sections 10A near the center of the plate 10 is a tab 30 extending from the lower edge 18 along a line of reduced thickness which functions as a hinge line 31 about which the tab 30 may be bent. The tab 30 is provided with holes 20A which preferably are countersunk.

As may be seen in Fig. 1, a femoral hip joint prosthesis P has been implanted in the femur F of a patient. Schematically in Fig. 1, the level of deficient bone illustrated by the line 21 in the area of the greater trochanter G of the patient is shown at a height sufficiently close to normal to receive screws 32 introduced in the holes 20. In contrast, however, the upper edge of the femur or level of deficient bone in the area of the lesser trochanter L has deteriorated to a point such that there is no bone available to receive screws introduced in the holes 20 in that area of the femur. Accordingly, the tab 30 has been bent around its hinge line 31 to engage the contour of the outer portion of the lesser trochanter L in order to permit a screw 32 to be affixed thereto. It should be recognized the use of screws in the areas between the lesser trochanter L and greater trochanter G will not be possible if the prosthesis is so close to the surface of the bone that the screws would contact the prosthesis. When thus affixed in position, the plate 10 provides needed support for the bone graft in the supratrochanteric region above the line 21.

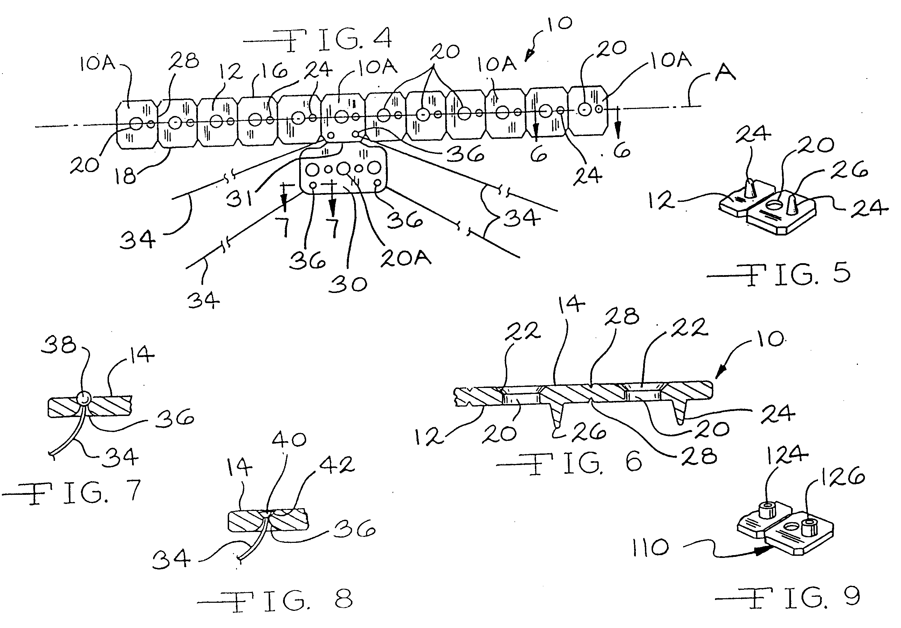

Referring specifically to Figs. 2, 4 and 7, there is provided a further embodiment in which a plurality of wires 34 may be utilized to provide additional support to the orthopaedic reconstruction plate 10 and to the portion of the femur F encircled by such wires 34. The wires 34 may be attached to the orthopaedic reconstruction please 10 by being threaded through apertures 36 in one or more of the sections 10A. As shown, the apertures 36 are in the center section 10A from which the tab 30 extends adjacent the transverse lines 28 of reduced thickness and the hinge line 31. If desired, four wires 34 may be utilized secured both to one of such sections 10A and to the tab 30. As may be readily seen in Fig. 7, the wire 34 may be retained in the aperture 36 by an enlarged head 38. In the alternate embodiment shown in Fig. 8, the wire 34 is provided with a flat head 40 which is received in a countersunk area 42 of the aperture 36. If desired, the flat head 40 could be welded or otherwise permanently adhered in the aperture 36.

In Fig. 2, only one pair of wires 34 extending from the tab 30 is utilized. The ends of the wires 34 may be joined together by tying a knot 44 or by utilizing a suitable retainer.

As will be appreciated, if the pins 24 were not present on the inner surface 12, the inner surface 12 would snugly abut and compress the bone when the screws 32 were affixed thereto. Such compression of the bone can have adverse effects on the vascularity of the femur particularly if the area under compression extends completely around the circumference of the bone. Accordingly, the presence of the pins 24, although providing localized compression in the areas contacted by the free ends 26, prevents excessive, potentially damaging compression from being imparted to the bone. The free end 26 of the pins 24 should be sufficiently blunt as to avoid significant penetration into the bone. The free ends may be either rounded or bluntly pointed.

In Fig. 9 there is shown a fragment of an alternate embodiment of orthopaedic reconstruction plate 110 in which the pin 124 is in the form of a short cylindrical wall having a flat end 126.

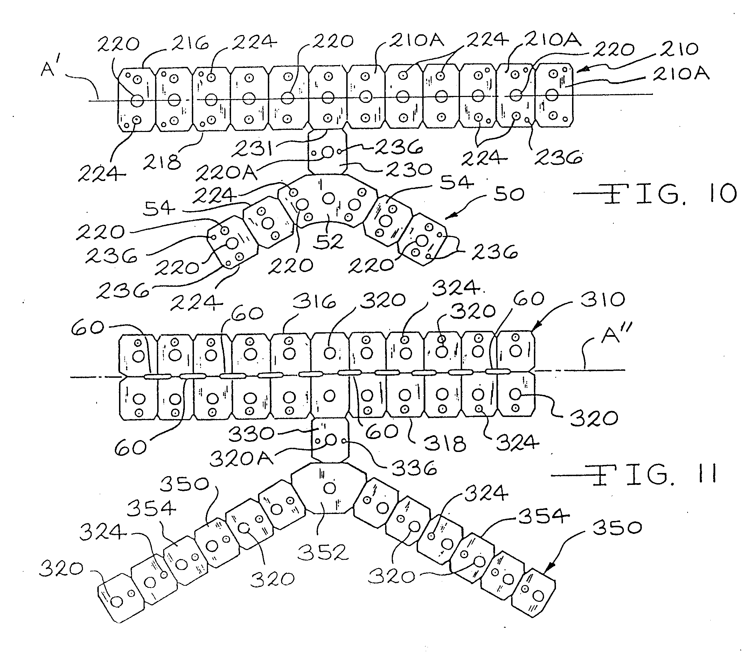

Referring now to Fig. 10, there is shown an alternate embodiment in which there is provided an orthopaedic reconstruction plate 210 having a tab 230 extending from the lower edge 218 of a centrally positioned section 210A, a hinge line 231 of reduced thickness. In this embodiment, although the holes 220 of sections 210A are centrally located along the axis A', the pins 224 is offset from such axis, with a pair of pins 224 being provided for each hole 220 and positioned on opposite sides thereof adjacent each of the upper edge 216 and lower edge 218. The tab 230 has a hole 220A and two apertures 236 but does not have any pins.

Under the embodiment of Fig. 10, an angled strip 50 having a central portion 52 extends from the tab 230. Extending from the central portion 52 are a pair of legs 54 each disposed at an angle tapering away from the axis A'. As is the case of the portion of the plate 210 extending along the axis A', the legs 54 are provided with holes 220 and pins 224 on opposite sides of the holes 220. Additionally, if desired, a number of wire receiving apertures 236 may be formed in various portions of the plate 210 including the angled strip 50. The central portion 52 is also provided with one or more holes 220 and pins 224 in the areas indicated.

Referring now to Fig. 11, there is shown another embodiment of orthopaedic reconstruction plate 310. Under this embodiment, the main body portion extends along an axis A'' and has two rows of apertures 320, one on each side of the axis A''. There are also provided two rows of pins 324, one adjacent each of the upper edge 316 and lower edge 318 and positioned between each such edge and the holes 320.

A plurality of slots 60 is formed in such plate 310 along the axis A'' to provide a line of weakness thus permitting the portions of the plate 310 on opposite sides of the axis A'' to be bent to follow the contour of the femur F in the area to which the hip revision plate 310 is to be attached.

A tab 330 having a hole 320A extends from a central portion of the lower edge 318. Preferably, under this embodiment, neither the tab 330 nor that portion of the plate 310 from which it extends is provided with pins. If desired, apertures 336 may be provided for receiving wires (not shown).

Extending from the tab 330 is an angled strip 350 having a central portion 352 integral with and extending from the tab 330. A pair of legs 354 extend outwardly from the central portion 352 and away from the lower edge 318. The legs 354 are provided with holes 320 and pins 324 which extend in a row centrally along each leg 354.

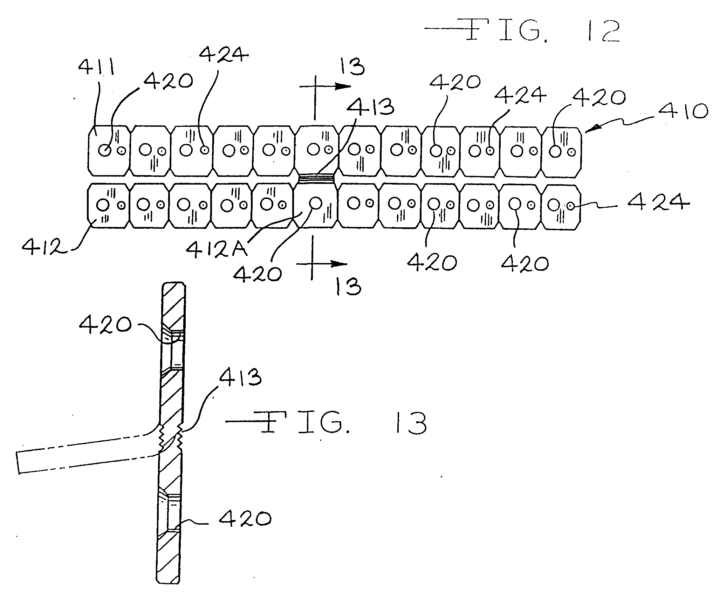

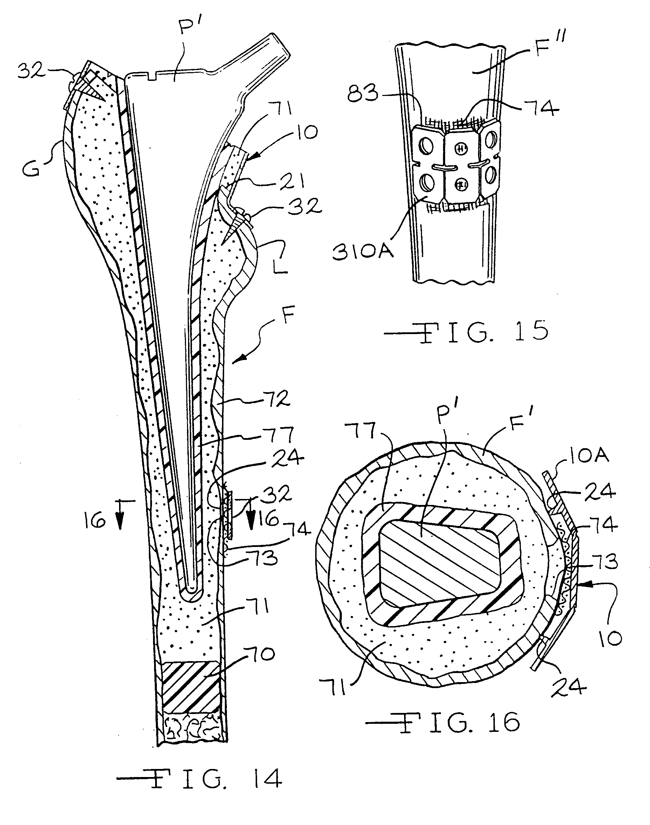

Referring now to Figs. 12 and 13, there is shown yet another embodiment in which there is provided an orthopaedic reconstruction plate 410 having a first elongated member 411 and a second elongated member 412 extending in parallel relationship thereto and joined to a central section 410 at a serration 413. The serration 413 provides a line of weakness permitting the second member 412 to be folded or bent thereabout to the contour of the bone. Each of the members 411 and 412 is provided with a series of holes 420 and a series of pins 424. Preferably, the section 412A of the second row 412 adjacent the serration 413 does not have a pin.

As can be seen in Fig. 3, the orthopaedic reconstruction plate 410 is secured to the femur F with the member 412 extending at an angle downwardly around a lower part of the femur than the upper portion 411 as a result of the orthopaedic reconstruction plate 410 being bent about the serrated area 413.

Although the orthopaedic reconstruction plate of the present invention is particularly useful in supporting graft material in the area of the proximal femur, it is also useful in supporting bone graft material in long bones, for example, in a central area of the femur or tibia or one of the bones of the arm.

Referring now to Figs. 14 and 16, there are shown sectional views of a femur F' showing a femoral hip joint prosthesis P' implanted in the intramedullary canal and showing an area having weak, deficient femoral cortices 72 in which the orthopaedic reconstruction plate 10 of the present invention has been used to retain crushed cancellous allograft 71 constituting the bone graft material.

The procedure for implanting the prosthesis P' includes preparing an enlarged cavity in the intramedullary canal of the femur F'. In revision surgery in particular, the cavity which is formed will be significantly larger than the prosthesis itself. Accordingly, it is desirable to place crushed cancellous bone allograft 71 in the enlarged cavity and to have it properly positioned in the intramedullary canal. Application Serial No. 07/735,524 filed July 23, 1991 which will issue as U.S. Patent No. 5,192,283 on March 9, 1993, of which I am a co-inventor, describes a method for compacting crushed cancellous allograft or bone material in the intramedullary canal in preparing the cavity to receive the hip joint prosthesis.

As shown in Fig. 14, a cement plug or restrictor 70 has been positioned at the bottom of the cavity as is well known in the art. Following placement of the restrictor 70, crushed cancellous allograft 71 is positioned in the cavity and may be compacted utilizing the cannulated tamp and method disclosed in Application Serial No. 07/735,524. Following compacting of the crushed cancellous allograft 71, the femoral hip joint prosthesis P' is placed in the prepared cavity with bone cement if the prosthesis is of a type intended for use with bone cement or without bone cement if the prosthesis is of a type intended to be used without such bone cement. In the example shown in Figs. 14-16 bone cement 77 is used. Thus, the compromised deficient femur is reconstructed by crushed cancellous allograft from inside the femur before cementing the femoral prosthesis in place. The reconstruction plate may be used with a femoral hip joint prosthesis which is collarless or with one having a collar.

As can be seen in Figs. 14 and 16, the femur includes the femoral cortices 72. However, there is also shown a bone defect 73 of weak, deficient femoral cortices. As a result of such weakness caused by the defect 73, it is likely that the crushed cancellous allograft 71 could escape through the defect 73 unless retained therein. This retention may be effected by using a short length of orthopaedic reconstruction plate of the present invention. As may be seen particularly in Fig. 16, the bone defect 73 is initially covered by a Dacron or biocompatible mesh 74. Then the orthopaedic reconstruction plate, for example, one or more of the sections 10A of the embodiment of plate 10 shown in Fig. 4 will be fastened with the pins 24 facing the bone of the femur F using screws 32.

Following positioning and compacting of the crushed cancellous allograft 71, the cement 77 and the prosthesis P' may be introduced into the prepared cavity.

Referring now to Fig. 15, there is shown a femur F'' having a defect 83 which is larger than the defect 73 shown in Fig. 14. In this event it may be desirable to use a section of a wider reconstruction plate of the type shown in Fig. 11 using one or more sections 310A.

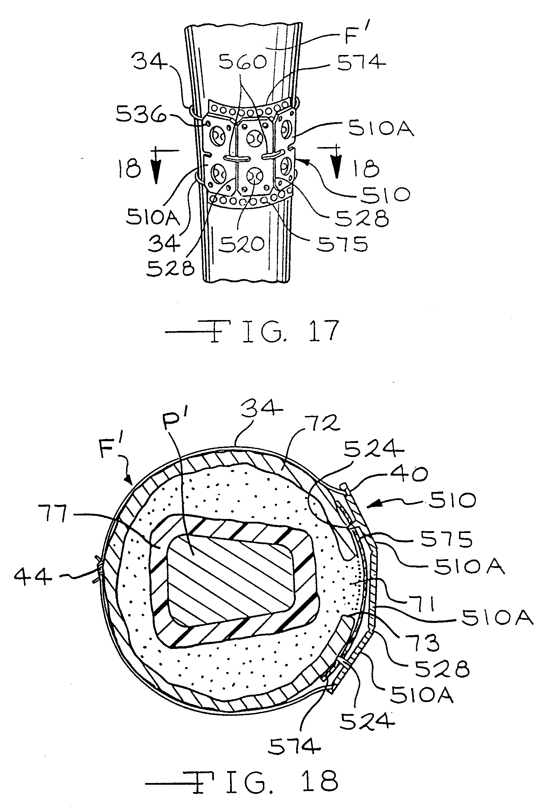

Referring now to Figs. 17 and 18 there is shown a fragmentary portion of a femur F' having weak, deficient cortices 72 with a defect 73 extending nearly or completely therethrough. A femoral hip joint prosthesis P' is shown as having been implanted in bone cement 77 with crushed cancellous allograft 71 used to promote bone growth in repairing the defect.

Under this embodiment, a reconstruction plate 510 is used. The reconstruction plate 510 is provided with a series of sections 510A joined along lines 528 of reduced thickness. Each of the sections 510A is provided with a pair of holes 520 in upper and lower areas thereof centrally positioned between the lines 528 of reduced thickness. Additionally, apertures 536 are provided to receive wires 34 and retain a head 40 secured thereto. Most of the sections 510A are provided with pins 524 extending from the side intended to face the bone to prevent excessive compressive forces from being imparted to the bone. However, it may be desirable to omit pins from the section 510A intended to overlie the defect 73. The plate 510 has a plurality of slots 560 extending therethrough at right angles to the lines 528 of reduced thickness and centrally positioned between the rows of holes 520.

Under this embodiment a mesh 574 formed of a biocompatible metal having a series of apertures 575 formed therein may be used. The mesh 574 is cut or otherwise shaped to a size larger than the size of the defect 73 in order to contain the allograft 71 from escaping through the defect 73 during and after its compaction in the intramedullary canal.

As may be seen particularly in Fig. 18, the reconstruction plate 510 is engaged to the femur F' along with the mesh 574 or immediately after by simply holding the mesh 574 in position. The pins 524 of the plate 510 extend through the apertures 575. The wire 34 is then wrapped around the femur F' and its ends are tied to form a knot 44 thus completing the procedure. With the reconstruction plate 510 firmly secured to the femur F', the crushed cancellous allograft 71 may be compacted in the intramedullary canal without being expelled through the defect. Following such compaction, the bone cement 77 and prosthesis P' may be positioned in the intramedullary canal.

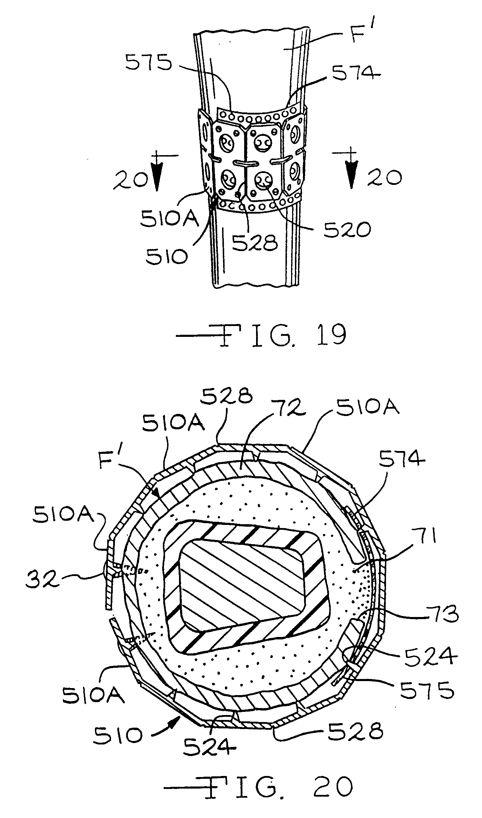

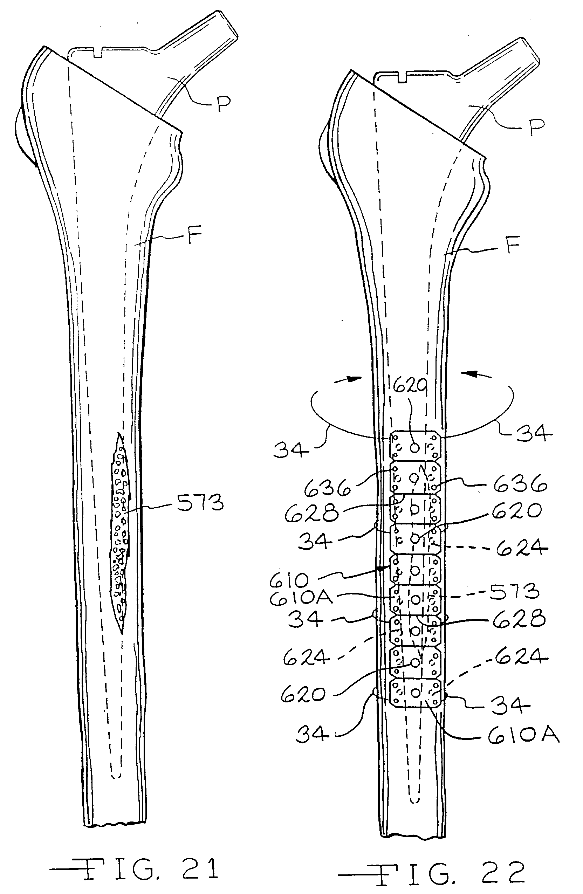

Referring now to Figs. 19 and 20, the femur F' has a defect 73 of a size similar to that shown in Figs. 17 and 18. As in that embodiment, the embodiment of Figs. 19 and 20 utilizes reconstruction plate 510; however, it has a sufficient number of sections 510A to permit its being wrapped nearly completely around the femur F'. Additionally, it is affixed by two or more screws 32 extending through the holes 520 and fastened to the femur F'. As in the previous embodiment, the pins 524 extends through apertures 575 of the mesh 574.

Referring now to Figs. 21 and 22, there is shown a femur F having a femoral hip joint prosthesis P, implanted therein. The femur F shown in Figs. 21 and 22 has been illustrated as having an elongated longitudinal or vertical defect 573. In repairing this defect, a reconstruction plate 610 consisting of a series of sections 610A joined along lines 628 of reduced thickness. Each section 610A has a centrally positioned hole 620, apertures 636 at each corner for receiving wires 34 and two pins 624, one on each side of the hole 620. A mesh (not shown) of Dacron® or metal may be used in the manner previously described. For this type of defect, it is preferable to retain the reconstruction plate 610 by the use of a series of wires 34 wrapped around the femur F and tied or otherwise joined together.

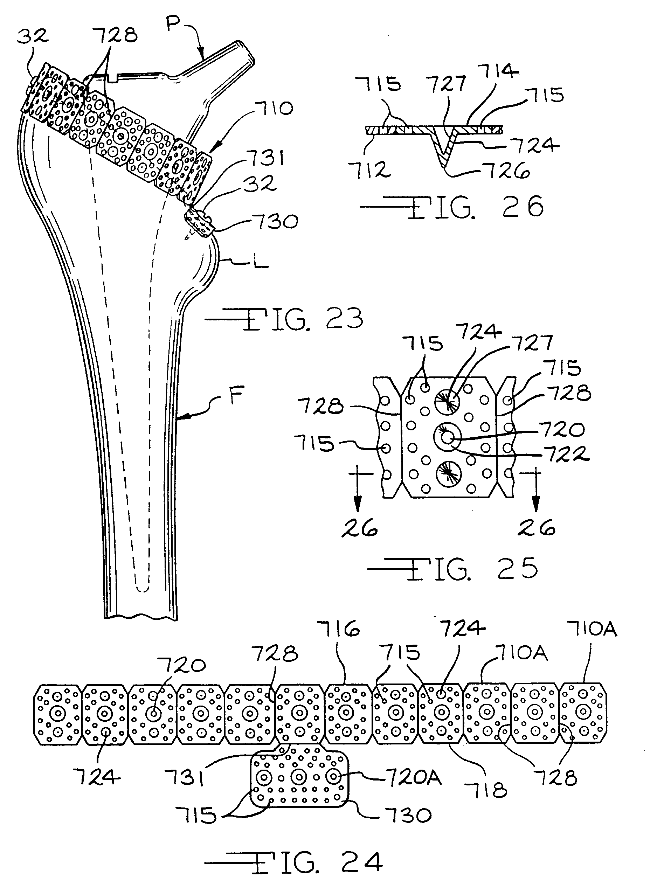

Referring now to Figs. 23-26, there is shown yet another embodiment of the present invention in which the reconstruction plate is formed from a surgical-grade metal or plastic having perforations throughout. Thus, in Fig. 23 there is shown a femur F having a femoral hip joint prosthesis P implanted therein. A reconstruction plate 710 consisting of a series of sections 710A are joined along lines 728 of reduced thickness. The plate 710 has an inner surface 712 (See Fig. 26) intended to face the bone when engaged to the femur F, an outer surface 714, an upper edge 716 and a lower edge 718. At least some and preferably all of the sections 710A are provided with a hole 720 which may be countersunk 722 similar to the plate 10 and holes 20 as shown in the embodiment of Figs. 1-6 in which the countersunk portion is shown at 22 in Fig. 6.

Under the present embodiment of Figs. 23-26, each of the sections 710A is provided with a plurality of perforations 715 extending therethrough from the inner surface 712 to the outer surface 714. Each of the perforations 715 has a diameter in the range of 1-3 mm with the performations being spaced apart approximately 1-5 mm throughout each of the sections 710A. The purpose of the perforations 715 located in multiple locations throughout each of the segments is to allow for the flow of blood and, thus, ensure that the reconstruction plate 710, when secured to the femur F or other bone does not interfere with circulation of blood to the bone.

An additional feature of the present embodiment permits the reconstruction plate to be manufactured at a significantly lower cost than the previous embodiments. As may be seen particularly in Figs. 25 and 26, there is provided a pin 724 which tapers to a free end 726. The pin 724 differs from the pins 24 of the embodiment of Figs. 1-6 in that it is not solid but rather has a hollowed-out portion or concavity 727 thus permitting it to be readily formed in a simple, rapid stamping operation. If desired, the perforations 715 may be formed in the same stamping operation.

The reconstruction plate 710 is shown as having a tab 730 similar to the tab 30 of the embodiment of Figs. 1-6. Preferably, the tab 730 has perforations 715. The plate 710 is of sufficient length to permit its being wrapped around the femur F with the end sections secured to the bone by one or more screws 32. The tab 730 bent about a hinge line 731 and is secured to the outer portion of the lesser trochanter L by one or more screws 32 extending through apertures 720A.

As in the previous embodiment, the pins 724 prevent excessive and damaging compressive force being imparted to the bone and provides benefit in preventing excessive interference with the flow of blood supply to the femur F. Under the present embodiment, the presence of the series of perforations 715 in multiple, closely spaced locations of each segment 710A allows for the flow of blood so that circulation of blood to the bone has even less likelihood of interference.

Although the present embodiment has been described in reference to the overall configuration of plate shown in Figs. 1 and 4 in which the plate is wrapped around the femur F, it should be understood that the features of this embodiment of (1) a plurality of closely spaced perforations in each of the sections and (2) the configuration of pin 724 which permits forming by a simple stamping operation may be utilized with the configurations of the plates shown in the other embodiments of the present invention.

Many other modifications will be readily apparent to those skilled in the art.

## Claims

1. An orthopaedic reconstruction plate comprising:
(a) an elongated body portion having inner and outer surfaces and upper and lower edges;
(b) a plurality of apertures in said body portion sized to receive fixation members;
(c) a plurality of pins extending from said inner surface; and
(d) a tab extending from said lower edge, said tab having at least one aperture.

2. The orthopaedic reconstruction plate of claim 1, wherein said tab extends from said lower edge along a line of juncture, said tab being foldable about said line of juncture.

3. The orthopaedic reconstruction plate of claim 1 further including at least two wires fixed to said body portion or said tab.

4. The orthopaedic reconstruction plate of claim 3, wherein said wires are permanently affixed to said body portion or said tab.

5. The orthopaedic reconstruction plate of claim 1, wherein said pins extend 1 to 3 mm from said inner surface.

6. The orthopaedic reconstruction plate of claim 1, wherein said body portion apertures are positioned substantially midway between said upper and lower edges.

7. The orthopaedic reconstruction plate of claim 6, wherein said pins are positioned to form two rows, one adjacent each of said upper and lower edges.

8. The orthopaedic reconstruction plate of claim 6, wherein said pins are positioned substantially midway between said upper and lower edges.

9. The orthopaedic reconstruction plate of claim 1, wherein said body portion is provided with a series of perforations substantially throughout, said perforations extending between said inner and outer surfaces.

10. The orthopaedic reconstruction plate of claim 9, wherein said perforations have diameters in the range of 1 to 3 mm and are separated from adjacent perforations by a distance in the range of 1 to 5 mm.

11. The orthopaedic reconstruction plate of claim 1, wherein said pins extend to a free end and taper from a relatively large cross-sectional size at said inner surface to a smaller cross-sectional size adjacent said free end and define a concavity with an opening facing said outer surface.

12. The orthopaedic reconstruction plate of claim 9, wherein said pins extend to a free end and taper from a relatively large cross-sectional size at said inner surface to a smaller cross-sectional size adjacent said free end and define a concavity with an opening facing said outer surface.

13. An orthopaedic reconstruction plate adapted to be attached to bone comprising a bendable elongated member having an inner surface adapted to face said bone, an outer surface, upper and lower edges, a plurality of apertures extending between said inner and outer surfaces and a plurality of pins extending from said inner surface to a free end.

14. The orthopaedic reconstruction plate of claim 13, wherein said pins have a length of 1 to 3 mm and said free end is blunt.

15. The orthopaedic reconstruction plate of claim 13, wherein said plate includes a plurality of sections and an area of reduced thickness between each of said sections, a majority of said sections having at least one of said pins and one of said apertures.

16. The orthopaedic reconstruction plate of claim 15, wherein at least some of said sections have an aperture and two of said pins positioned such that said aperture is located therebetween.

17. The orthopaedic reconstruction plate of claim 15 further including at least two wires fixed to at least one of said sections.

18. The orthopaedic reconstruction plate of claim 15 further including at least two wires fixed to each of at least two of said sections.

19. The orthopaedic reconstruction plate of claim 13, wherein said member is provided with a series of perforations substantially throughout, said perforations extending between said inner surface and said outer surface.

20. The orthopaedic reconstruction plate of claim 19, wherein said perforations have diameters in the range of 1 to 3 mm and are separated from adjacent perforations by a distance in the range of 1 to 5 mm.

21. The orthopaedic reconstruction plate of claim 13, wherein said pins extend to a free end and taper from a relatively large cross-sectional size at said inner surface to a smaller cross-sectional size adjacent said free end and define a concavity with an opening facing said outer surface.

22. The orthopaedic reconstruction plate of claim 19, wherein said pins extend to a free end and taper from a relatively large cross-sectional size at said inner surface to a smaller cross-sectional size adjacent said free end and define a concavity with an opening facing said outer surface.

23. An orthopaedic reconstruction device intended to be affixed to a bone comprising, in combination:
(a) a metal mesh having a plurality of apertures extending across a major portion thereof; and
(b) a plate having inner and outer surfaces, a plurality of pins extending from said inner surface sized to fit in said apertures, at least a portion of said pins extending through said apertures and means for fastening said plate to bone.

24. The orthopaedic reconstruction device of claim 23, wherein said pins extend 1 to 3 mm from said inner surface.

25. The orthopaedic reconstruction device according to claim 23, wherein said means for fastening said plate to bone comprises holes in said plate and a plurality of screws extending through said holes.

26. The orthopaedic reconstruction device according to claim 23, wherein said means for fastening said plate to bone comprises a plurality of wires each having a first end engaged to said plate and a second end, said wires having a length permitting engagement of said second end of one of said wires to said second end of another of said wires.

27. The orthopaedic reconstruction device according to claim 23, wherein said plate includes a plurality of sections, and an area of reduced thickness between each of said sections, a majority of said sections having said pins.

28. The orthopaedic reconstruction device of claim 23 wherein said plate has upper and lower edges joining said inner and outer surfaces and further including a tab extending from said lower edge.

29. The orthopaedic reconstruction device of claim 28, wherein said tab has inner and outer surfaces, at least one aperture extending through said tab from said inner surface to said outer surface and at least one pin extending from said inner surface.

30. The orthopaedic reconstruction device of claim 28 wherein said plate includes a second elongated member having a central portion extending from said tab and first and second end portions extending in opposite directions from said tab, said second elongated member having inner and outer surfaces and a plurality of pins extending from said inner surface.

31. The orthopaedic reconstruction device of claim 30, wherein said first and second end portions taper away from said lower edge as they extend from said tab.

32. The orthopaedic reconstruction device of claim 23, wherein said plate portion is provided with a series of perforations substantially throughout, said perforations extending between said inner and outer surfaces.

33. The orthopaedic reconstruction device of claim 32, wherein said perforations have diameters in the range of 1 to 3 mm and are separated from adjacent perforations by a distance in the range of 1 to 5 mm.

34. The orthopaedic reconstruction device of claim 23, wherein said pins extend to a free end and taper from a relatively large cross-sectional size at said inner surface to a smaller cross-sectional size adjacent said free end and define a concavity with an opening facing said outer surface.

35. The orthopaedic reconstruction device of claim 32, wherein said pins extend to a free end and taper from a relatively large cross-sectional size at said inner surface to a smaller cross-sectional size adjacent said free end and define a concavity with an opening facing said outer surface.

36. A method for repairing deficient cortices in a bone comprising the steps of:
(a) providing an orthopaedic reconstruction plate having an inner surface with a plurality of pins extending therefrom to a free end and apertures for receiving retaining screws;
(b) affixing said plate to said bone in the area of said deficient cortices with said free ends of said pins contacting said bone;
(c) placing crushed cancellous allograft in said deficient cortices; and
(d) retaining said crushed cancellous allograft with said plate.

37. A method of reconstructing a femur having deficient bone material at a level of the trochanteric region comprising the steps of:
(a) providing an orthopaedic reconstruction plate formed of a flexible strip of biocompatible material, said strip having first and second surfaces and upper and lower edges between said first and second surfaces, said first surface having a plurality of pins extending therefrom;
(b) wrapping said plate around the femur in said trochanteric region such that at least a portion of said pins contact the bone and such that said upper edge extends above at least a portion of said level; and
(c) fastening said plate to said femur in said trochanteric region.

38. The method according to claim 37, wherein said plate is provided with a plurality of holes and said step of affixing comprises affixing screws through at least some of said holes.

39. The method according to claim 38, wherein said affixing step comprises applying at least one screw through a hole in the trochanteric region adjacent the greater trochanter and applying at least one screw through one of said holes into the lesser trochanter.

40. The method according to claim 38, wherein said plate is provided with a tab extending from said lower edge and a plurality of holes and said affixing step includes applying at least one screw through a hole in the trochanteric region adjacent the greater trochanter and bending said tab into conformity with the lesser trochanter and affixing a screw through a hole in said tab into said lesser trochanter.

41. The method according to claim 40 further including the step of wrapping a further support member around said femur from an area adjacent said lesser trochanter to an area distally spaced from said greater trochanter.
